# EUROPEAN PATENT SPECIFICATION

(11) **EP 3 302 289 B1**
(45) Date of publication and mention of the grant of the patent: **13.01.2021**
(21) Application number: 16731970.6
(22) Date of filing: 01.06.2016
(51) Int. Cl.: A61B 10/00, A61K 39/04, A61B 10/02

(54) **METHOD AND DEVICE FOR COLLECTING MYCOBACTERIUM COMPLEX SPECIES FROM THE ORAL CAVITY OF A PATIENT**
VERFAHREN UND VORRICHTUNG ZUM SAMMELN VON MYCOBACTERIUMSKOMPLEXSPEZIES AUS DER MUNDHÖHLE EINES PATIENTEN
PROCÉDÉ ET DISPOSITIF POUR COLLECTER DES ESPÈCES DU COMPLEXE MYCOBACTERIUM DEPUIS LA CAVITÉ BUCCALE D'UN PATIENT

(30) Priority: 01.06.2015 ZA 201503932
(43) Date of publication of application: 11.04.2018
(73) Proprietor: North-West University, 2531 Potchefstroom (ZA); Stellenbosch University, 7600 Stellenbosch (ZA); University of Nebraska Lincoln, Lincoln, Nebraska 68588 (US)
(72) Inventor: GROBLER, Anne, Frederica, 2520 Potchefstroom (ZA); CRONJE, Lizl, 7600 Stellenbosch (ZA); VILJOEN, Hendrik, Lincoln, Nebraska 68588 (US)
(74) Representative: Cohausz & Florack
(86) International application number: PCT/IB2016/053212
(87) International publication number: WO 2016/193918

(56) References cited:
- EP-A1- 1 338 886
- EP-A1- 2 868 339
- WO-A1-2010/055460
- WO-A1-2013/035062
- WO-A1-2013/088396
- GB-A- 2 457 654
- US-A1- 2004 162 500
- US-A1- 2010 279 271

## Description

### FIELD OF THE INVENTION

This invention relates to a method and device for collecting *Mycobacterium* complex species, particularly, but not exclusively, to a method and device for collecting *Mycobacterium tuberculosis* (MTB) from the oral cavities of children and infants. The invention further relates to a method of preparing biological material obtained from such collected sample.

### BACKGROUND TO THE INVENTION

Tuberculosis (TB) is a disease caused by MTB and is responsible for approximately 1.4 million deaths annually and it disproportionately affects developing countries, particularly sub-Saharan Africa. Notwithstanding technical advances, expanded programs and significant expenditures, the disease is still not under control.

Despite being a preventable and treatable disease, TB remains a leading cause of morbidity and mortality worldwide. It is estimated that one third (2.9 million) of all TB cases are not notified to the health systems. This gap is in part due to missed diagnosis; diagnostics account for only 2% of the cost of health care, but affect 60-70% of treatment decisions [1].

The current methods and tools for detecting TB are focussed on the adult population. These diagnostics include antibody based methods, PCR based methods using sputum samples, bronchial washings and urine samples. Some additional methods include mass spectrometry to detect mycolic acids, and a gas sensor to detect different species of *Mycobacteria.* Some devices for mouth swabs have also been proposed, but without apparent success and/or efficacy.

WO2013/088396A1 provides an apparatus and method for collecting biological material. This apparatus and method is however based on gastric aspiration or gastric lavage which is fairly encompassing to the patient and causes substantial discomfort and inconvenience to the patient, especially children. In addition, there are some associated disadvantages regarding accurate diagnosis from biological material in the gastric environment.

WO2013/035062A1 discloses a method of preparing biological material from a biological sample in order to perform the subsequent diagnosis. However, this does not overcome associated problems of obtaining the said biological material in a convenient and effective manner that would result in effective diagnosis.

Despite the existing means alluded to above, there remains a lack of a sufficiently sensitive tool to detect early disease in adults, paucibacillary disease in HIV infected adults and TB in all children. Young children and HIV infected adults are particularly difficult to diagnose; obtaining adequate sputum from young children requires relatively invasive and resource-intensive procedures.

Children respond differently to a TB challenge than adults; their immunological response is not the same and the disease presents with different pathology. The lymph plays a key role in the immunological response and it is conceivable that bacilli (free or phagocytised) may traffic via the lymphatic system [2]. Children with TB typically present with enlarged mediastinal and hilar lymph nodes. An example of this patho-physiological response is scrofula. The salivary glands, particularly the parotid, have embedded lymph nodes; nodes are also located close to the submaxilliary gland.

The MTB bacilli may also be present in the oral cavity as a result of transfer from the lungs into the oral cavity through coughing or sneezing. The collection of salivary specimens from the oral cavity to test for the presence of bacilli is accordingly a procedure for the diagnosis of tuberculosis and may also contribute to existing knowledge of TB pathology in children.

Current methods to collect respiratory specimens for TB investigation from young children are relatively invasive, including procedures such as gastric aspiration, sputum induction with nasopharyngeal suctioning or bronchoalveolar lavage. In many settings the diagnosis is made clinically, oftentimes by nurses or doctors not necessarily trained in paediatric TB.

The accurate diagnosis of TB in young children requires in addition that the specimen collection methods are also improved in order to provide samples with adequate bacteriological yield. As most of the laboratory techniques used for the diagnosis of TB are dependent on the detection of MTB, the pathogen responsible for the disease, it is of utmost importance that good quality specimens are obtained with the highest possible concentration of *Mycobacteria* to enable the accurate diagnosis of TB. In the current era of multi drug-resistant

(MDR)/extensively drug-resistant (XDR)/totally drug-resistant (TDR)-TB, there is also a growing need to isolate the bacillus in order to perform drug susceptibility testing. It is therefore vital to improve the specimen collection method in children in order to provide samples with adequate bacteriological yield for fast and accurate detection and timely treatment.

New TB diagnostics, which are simple, sensitive, rapid and which use culturally acceptable biological samples, are crucial to curb the global TB epidemic.

### OBJECT OF THE INVENTION

It is accordingly an object of the current invention to provide a method and device for collecting *Mycobacterium* complex species, such as *Mycobacterium tuberculosis* with which the abovementioned disadvantages could at least partially be alleviated or overcome and to provide a method of preparing biological material from such collected sample.

### SUMMARY OF THE INVENTION

The invention is defined in claims 1, 4, 8, 9, 12. Further aspects and preferred embodiments are defined in the appended claims. Aspects, embodiments and examples of the present disclosure which do not fall under the scope of the appended claims do not form part of the invention and are merely provided for illustrative purposes.

According to a first aspect of the invention there is provided a device (10) for collecting a biological sample from the oral cavity of a patient, comprising a body having a distal end and an operative end portion (14), at least the operative end portion being manufactured from a parent polymer which has been functionalised with a chemical compound having an affinity to a *Mycobacterium* complex species including *Mycobacterium tuberculosis* to collect and operatively detect the presence of a *Mycobacterium* complex species in the sample, characterised in that the operative end portion being in the form of a pacifier (14B) or a brush (14A) having bundled strands and the parent polymer is functionalised with either one or more of Concanavalin A and a positive charged ammonium moiety and a hydrocarbon chain, and wherein the Concanavalin A and the positive charged ammonium moiety and hydrocarbon chain is attached to the parent polymer covalently or electrostatically.

The invention provides for the parent polymer to be selected from any one or more of the group consisting of silicon based polymers, polyester based polymers and polyurethane based polymers.

The hydrocarbon chain may be provided as a C₁₀, C₁₂ or C₁₄ hydrocarbon chain. The distal end may be in the form of a handle. The invention provides for the *Mycobacterium* complex species to include *Mycobacterium tuberculosis.*

The invention yet further provides for the patient to include a child or infant.

According to a second aspect of the invention there is provided a method of collecting a *Mycobacterium* complex species present in a biological sample from the oral cavity of a patient comprising:
- providing a parent polymer in a device (10) with a body having a distal end and an operative end portion (14), at least the operative end portion the operative end portion being in the form of a pacifier (14B) or a brush (14A) having bundled strands and being manufactured from the parent polymer;
- the parent polymer is functionalised with either one or more of Concanavalin A and a positive charged ammonium moiety and a hydrocarbon chain, and wherein the Concanavalin A and the positive charged ammonium moiety and hydrocarbon chain is attached to the parent polymer covalently or electrostatically having an affinity to the *Mycobacterium* complex species;
- bringing the functionalised polymer into contact with the inside walls of the oral cavity of the patient; and
- removing said functionalised polymer from the oral cavity of the patient.

The parent polymer may be selected from any one or more of the group consisting of silicon based polymers, polyester based polymers and polyurethane based polymers.

The step of functionalising the parent polymer may include the step of attaching the chemical compound to the parent polymer covalently or electrostatically.

The invention provides for adding a flavouring agent to the polymer to make collecting the biological sample more acceptable to the patient.

In accordance with a third aspect of this invention, there is provided the use of the device or method, as described hereinbefore, for collecting a biological sample having a *Mycobacterium* complex species from the oral cavity of a patient for diagnosis of Tuberculosis.

In accordance with a fourth aspect of this invention, there is provided a method for collecting *Mycobacterium* complex species present in a biological sample and preparing biological material therefrom with the device according to a first aspect of the invention comprising the steps of:
A. collecting the *Mycobacterium* complex species according to the second aspect of the invention to form biological material characterised in that the functionalised polymer forms a capturing scaffold that operatively collects the *Mycobacterium* complex species from the oral cavity of the patient; and
B. preparing the biological material including the steps of altering the constitutive characteristics of the sample in the presence of the capturing scaffold by concomitantly:
   - adding a lysis buffer containing a solubilising agent, a detergent and preferably a reducing agent to the sample;
   - and concomitantly physically treating through agitation and elevation of the temperature of the biological sample above 40 degrees Celsius and up to 100 degrees Celsius, preferably 92 degrees Celsius, for simultaneously:
   - inhibiting coagulation of the sample;
   - lysing the sample to release the biological material from the sample, and make the biological material available; and
   - capturing at least one fraction of the biological material on the capturing scaffold.

Subsequently the capturing scaffold, with the particular fraction of biological material captured thereon, is removed from the remainder of the sample and preferably washed with de-ionised water. The capturing scaffold is subsequently stored, transported or presented for analysis of the biological sample.

According to a fifth aspect of the invention there is provided a kit for use in a method of collecting *Mycobacterium* complex species present in a biological sample and preparing biological material therefrom, the kit comprising:
- a device for collecting the biological sample as described hereinbefore, the device forming a capturing scaffold; and
- a container including a lysis buffer containing a solubilising agent, a detergent and preferably a reducing agent for operatively simultaneously lysing the biological sample to make the biological sample available, inhibiting coagulation of the biological sample, and capturing at least one fraction of the biological material on the capturing scaffold.

### BRIEF DESCRIPTION OF THE ACCOMPANYING DRAWINGS

The invention is now described by way of non-limiting examples with reference to the accompanying drawings wherein:
- figure 1:: is a perspective view of a device according to a first embodiment of the invention;
- figure 2:: is a perspective view of a device according to a second embodiment of the invention; and
- figure 3:: is a fluorescence microscopy image showing captured bacilli visible on the surface of a functionalised parent polymer.

### DESCRIPTION OF PREFERRED EMBODIMENTS OF THE INVENTION

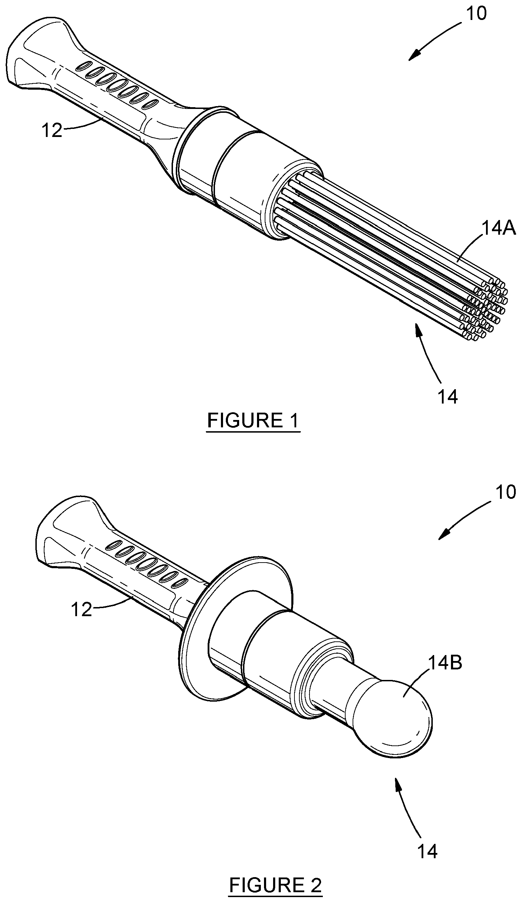

Referring to figure 1, a device according to a first preferred embodiment of the invention, for collecting *Mycobacterium tuberculosis* (MTB) from the oral cavities of children and infants, is generally designated by numeral 10.

The device 10 has a body provided with a distal end in the form of a handle 12 which can be manipulated by the user during use as will be explained below; and an operative end portion 14 which, in the embodiment shown in figure 1, is in the form of bundled strands of fibres flared into a brush 14A.

In the alternative embodiment shown in figure 2, the operative end portion 14 is in the form of a pacifier 14B.

The brush 14A and pacifier 14B consists of a parent polymer functionalised with a variety of chemical compounds/ligands to aid the binding/capture of microbes, more specifically mycobacteria and even more specifically *Mycobacterium tuberculosis* (MTB). Two chemical compounds/ligands are used namely Concanavalin A (a carbohydrate-binding protein) and a chemical compound with a positive charged ammonium moiety and a hydrocarbon chain ("ConA" and "Cx"). It will be understood that the Cx may include a C₁₀, C₁₂ or C₁₄ hydrocarbon chain.

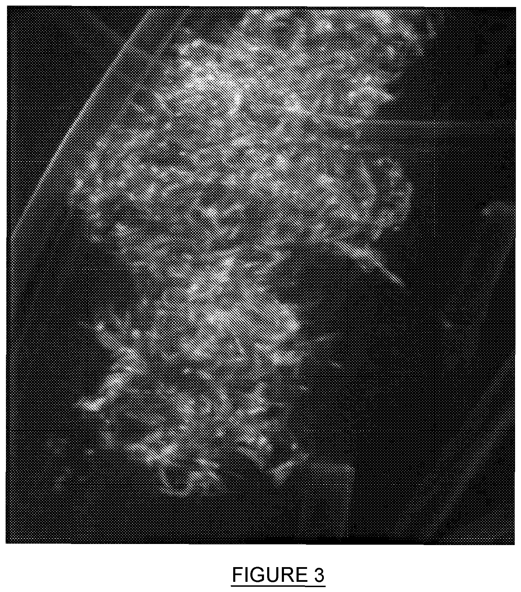

Figure 3 illustrates the fluorescence microscopy image of a modified polymer (functionalised with Cx), in this instance using the parent polymer styrene-co-maleic anhydride (SMA), after incubation with an attenuated mycobacterial strain at 37 °C for one hour. The captured bacilli are visible on the surface of the modified polymer.

For the brush 14A, a modified polyester-based polymer will be used as parent polymer, functionalised with ConA and Cx. The brush is injection moulded and the handle 12 is accordingly made from the functionalised polymer, although the handle 12 can be made of any other suitable plastic material.

Pacifiers 14B are manufactured using silicone- or polyester-based polymers that are safe, non-toxic and inert. The pacifiers 14B will be functionalised with the two chemical compounds mentioned previously. The pacifiers 14B are injection moulded using the functionalised polymer.

Both ConA and Cx functionalisation agents will be attached to the parent polymer covalently or electrostatically to prevent leaching when applied to the environment in the oral cavity.

Flavouring may also be added to the polymer which will make the collection process more acceptable to patients.

In use, the device 10 is simply inserted into the oral cavity to bring the device into contact with the inside walls of the oral cavity of the patient and removed therefrom to collect a biological sample for further preparation and processing. The samples are further prepared, processed and analysed by smear microscopy, culture, GeneXpert or any other suitable method known in the art to detect the presence of *Mycobacterium* complex species. Preparation of the biological sample will specifically by done by the molecular assay as described in the co-applicant's PCT/IB2012/054608, the contents of which is incorporated fully herein. The device 10 can be fitted directly into the the lysis micro reactor ('LMR') or processing device (not shown) disclosed by PCT/IB2012/054608 and the handle 12 furthermore is moulded to a diameter that will serve as a lid for the mentioned LMR device. In this instance, the operative end portion 14, forms the capturing scaffold as described below.

Preparing biological material from the biological sample in the form of the sputum sample obtained by the steps mentioned above will now be described.

The method of preparing biological material includes the steps of altering the constitutive characteristics of the sample in the presence of the capturing scaffold by concomitantly:
- adding a lysis buffer containing a solubilising agent, a detergent and a reducing agent to the sample;
- elevating the temperature of the sample above 40 degrees Celsius and up to a 100 degrees Celsius, preferably 92 degrees Celsius; and
- physically treating the sample through agitation,
for simultaneously:
- inhibiting coagulation of the sample;
- lysing the sample to release the biological material from the sample, thus making the biological material available; and
- capturing at least one fraction of the biological material on the capturing scaffold.

Subsequently the capturing scaffold, with the particular fraction of biological material captured thereon, is removed from the remainder of the sample and washed with de-ionised water. The capturing scaffold is subsequently stored, transported or presented for analysis of the biological sample.

Purified starch such as purified corn or potato starch is optionally added to the biological material simultaneously with the lysis buffer for neutralising any PCR inhibitors that may be present in the biological material.

The solubilising agent comprises a chaotropic salt in the lysis buffer. The lysis buffer is selected from the group consisting of phosphate buffers, 4-(2-hydroxyethyl)-1-piperazineethanesulfonic acid (HEPES), N-Cyclohexyl-2-aminoethanesulfonic acid (CHES), *N*-cyclohexyl-3-aminopropanesulfonic acid (CAPS) and piperazine-*N,N*-bis(2-ethanesulfonic acid) (PIPES), Tris-HCI, as well as other tris(hydroxymethyl)aminomethane (Tris) buffers containing ethylene diamine tetra-acetic acid (EDTA), ethylene glycol tetra-acetic acid (EGTA), deoxycholate, sodium chloride (NaCI), sodium phosphate, octylphenoxypolyethoxyethanol, and non-ionic surfactants provided with a hydrophilic polyethylene oxide group and a hydrocarbon lipophilic or hydrophobic group, and combinations thereof. The lysis buffer has a pH of between 4 and 9, preferably 7.5.

The chaoropic salt is selected from the group consisting of urea, thiourea, guanidine hydrochloride, lithium perchlorate, sodium iodine, sodium perchlorate, guanidine isothiocyanate, guanidine carbonate, guanidine thiocyanate, derivatives thereof, preferably guanidine hydrochloride and combinations thereof.

The detergent is selected from the group consisting of 3-[(3-cholamidopropyl) dimethylammonio]-1-propanesulfonate (CHAPS), a nonionic surfactant and emulsifier derived from polyethoxylated sorbitan and oleic acid, a nonionic surfactant which has a hydrophilic polyethylene oxide group and a hydrocarbon lipophilic or hydrophobic group, saponin, sodium deoxycholate, SDS, octyl glucoside, octyl thioglucoside, laurly maltose, octylphenoxypolyethoxyethanol, and combinations thereof.

The reducing agent is selected from the group consisting of 2-mecarptoethanol, dithiothreitol (DTT), 2-mercaptoethylamine, Tris(2-carboxyl)phosphine (TCEP), cysteine HCI, *N*-ethylmaleimide, Nacystelyn, dornase alfa, thymosin β₄, guaifenesin TCEP HCI, and combinations thereof.

The prepared biological material captured on the capturing scaffold is subsequently air dried and stored at ambient temperature. The capturing scaffold is stored, transported or presented for analysis of the biological sample.

The invention accordingly provides a functionalised polymer which specifically binds to mycolic acids, the hallmark component of mycobacterial cell walls. It also utilizes the negative charge of the mycobacterial cell wall to bind electrostatically via the positive charge on the polymer backbone.

Collecting clinical specimens from the oral cavities of infants and children with either the brushes 14A or pacifiers 14B as described for the diagnosis of TB is accordingly a viable, less invasive alternative to current methods and provides a feasible approach for collecting biological samples at home by parents or at schools and clinics under supervision of teachers and nurses.

The use of the invention to scavenge the oral cavity makes the process less invasive, more accessible and less stigmatising to the broader public. In the case of infants, the use of a pacifier is proposed.

The use of brushes and pacifiers that is made of a material that has high affinity for binding specifically MTB complex and which is safe for use in humans is accordingly proposed. This has never been tested or suggested as a diagnostic procedure for TB. This method and device for specimen collection would accordingly remove a major barrier to TB diagnosis in children, by avoiding the need for time-consuming, resource-intensive and relatively invasive procedures for sputum collection or obtaining gastric aspirates.

The invention also provides for a method and device which provides biological samples with adequate bacteriological yield. The capturing of the mycobacteria on a limited surface acts as a concentration step for mycobacteria in a sample. Such a concentration step increases the number of mycobacteria/volume of sample obtained.

Yet further, for sputum-scarce adults, brushings collected from the oral cavity may also aid in improving diagnosis. The simplicity of this invention would enable the collection technique to be rolled out at point-of-care and even community level. It could potentially become an effective screening test for the early detection of TB disease, and it could have major impact on global TB control.

A simple, non-invasive specimen collection method coupled with a highly sensitive assay results in early detection of TB disease and will accordingly impact on TB transmission in communities as well as reduce TB-related morbidity and mortality among children, who are most difficult to diagnose using current available methods. The impact will broaden significantly if the system performs well with adults - this specimen collection method will be non-stigmatizing and enjoy better cultural acceptance, and it could open the way to screening for TB.

The invention provides a simple, culturally acceptable and highly sensitive tool to detect early TB disease in adults and diagnose paucibacillary disease in children. This tool is also robust, safe for use by the patient and caregiver, as well as cost effective, since TB is a disease of the developing world.

The invention yet further provides an efficient and robust method for preparing uncontaminated biological material from the collected sputum sample without applying numerous consecutive steps that necessarily have to be taken in a laboratory environment or in different chambers of the LMR. It was found that, in particular, a kit comprising the said capturing scaffold strip and lysis buffer as described above could be prepared and provided to field workers collecting samples in rural areas using the LMR. The sample could be combined with the scaffold and lysis buffer in the LMR and the scaffold strip removed once the biological material has been captured on the scaffold strip. The advantages of this single step method over the relatively complex prior art methods requiring a laboratory environment, numerous enzymes and other reagents and multiple steps, are evident.

It is accordingly asserted that the disadvantages associated with known methods and devices for collecting *Mycobacterium* complex species could be alleviated with the method and device according to the invention.

It will be understood that the above examples are not to be interpreted as limiting the scope of the invention. For example, it must be understood that the use of functionalised polymers to capitalise on specific moieties expressed on cell exteriors is not limited to the above example and may find wider application without departing from the scope of the invention. The *Mycobacterium* complex species may furthermore include those associated with Leprosy disease.

Similarly, the mentioned parent polymers and chemical compounds having affinity to *Mycobacterium* is not to be limited to those mentioned herein.

### REFERENCES

[1] BRINGING THE LAB TO THE PATIENT: developing point-of-care diagnostics for resource limited settings. A REPORT FROM THE AMERICAN ACADEMY OF MICROBIOLOGY. 2012.
[2] Behr, MA and WR Waters, Is tuberculosis a lymphatic disease with a pulmonary portal? www.thelancet.com/infection Published online November 22, 2013 http://dx.doi.org/10.1016/S1473-3099(13)70253-6.

## Claims

1. A device (10) for collecting a biological sample from the oral cavity of a patient, comprising a body having a distal end and an operative end portion (14), at least the operative end portion being manufactured from a parent polymer which has been functionalised with a chemical compound having an affinity to a *Mycobacterium* complex species including *Mycobacterium tuberculosis* to collect and operatively detect the presence of a *Mycobacterium* complex species in the sample, **characterised in that** the operative end portion being in the form of a pacifier (14B) or a brush (14A) having bundled strands and the parent polymer is functionalised with either one or more of Concanavalin A and a positive charged ammonium moiety and a hydrocarbon chain, and wherein the Concanavalin A and the positive charged ammonium moiety and hydrocarbon chain is attached to the parent polymer covalently or electrostatically.

2. The device according to claim 1, wherein the parent polymer is selected from any one or more of the group consisting of silicon based polymers, polyester based polymers and polyurethane based polymers.

3. The device according to claim 2, wherein the hydrocarbon chain is a C₁₀, C₁₂ or C₁₄ hydrocarbon chain.

4. A method of collecting a *Mycobacterium* complex species present in a biological sample from the oral cavity of a patient, comprising:
- providing a parent polymer in device (10) with a body having a distal end and an operative end portion (14), at least the operative end portion the operative end portion being in the form of a pacifier (14B) or a brush (14A) having bundled strands and being manufactured from the parent polymer;
- the parent polymer is functionalised with either one or more of Concanavalin A and a positive charged ammonium moiety and a hydrocarbon chain, and wherein the Concanavalin A and the positive charged ammonium moiety and hydrocarbon chain is attached to the parent polymer covalently or electrostatically having an affinity to the *Mycobacterium* complex species;
- bringing the functionalised polymer into contact with the inside walls of the oral cavity of the patient; and
- removing said functionalised polymer from the oral cavity of the patient.

5. The method according to claim 4, wherein the parent polymer is selected from any one or more of the group consisting of silicon based polymers, polyester based polymers and polyurethane based polymers.

6. The method according to claim 5, wherein the hydrocarbon chain is a C₁₀, C₁₂ or C₁₄ hydrocarbon chain.

7. The device or method according to any one of the preceding claims, wherein a flavouring agent is added to the parent polymer to make collecting the biological sample more acceptable to the patient.

8. Use of the device or method according to any one of the preceding claims, for collecting a biological sample having a *Mycobacterium* complex species from the oral cavity of a patient for diagnosis of Tuberculosis.

9. A method for collecting *Mycobacterium* complex species present in a biological sample and preparing biological material therefrom with the device of claims 1 to 3, comprising:
A. collecting the *Mycobacterium* complex species according to any one of claims 4 to 6 to form biological material **characterised in that** the functionalised polymer forms a capturing scaffold that operatively collects the *Mycobacterium* complex species from the oral cavity of the patient; and
B. preparing the biological material including the steps of altering the constitutive characteristics of the sample in the presence of the capturing scaffold by concomitantly:
- adding a lysis buffer containing a solubilising agent, a detergent and preferably a reducing agent to the sample;
- and concomitantly physically treating through agitation and elevation of the temperature of the biological sample above 40 degrees Celsius and up to 100 degrees Celsius, preferably 92 degrees Celsius, for simultaneously:
- inhibiting coagulation of the sample;
- lysing the sample to release the biological material from the sample, and make the biological material available; and
- capturing at least one fraction of the biological material on the capturing scaffold.

10. The method according to claim 9, including the subsequent step of removing the capturing scaffold, with the particular fraction of biological material captured thereon, from the remainder of the sample and preferably washing the capturing scaffold with de-ionised water.

11. The method according to claim 10, including the subsequent step of storing, transporting or presenting the biological sample for analysis.

12. A kit for use in a method of collecting *Mycobacterium* complex species present in a biological sample and preparing biological material therefrom, the kit comprising:
- a device for collecting the biological sample according to any one of claims 1 to 3, the device forming a capturing scaffold that operatively collects the biological sample from the oral cavity of the patient; and
- a container including a lysis buffer containing a solubilising agent, a detergent and preferably a reducing agent for operatively simultaneously lysing the biological sample to make the biological sample available, inhibiting coagulation of the biological sample, and capturing at least one fraction of the biological material on the capturing scaffold.

## Patentansprüche

1. Eine Vorrichtung (10) für die Entnahme einer biologischen Probe aus der Mundhöhle eines Patienten, welche einen Grundkörper mit einem distalen Ende und einem aktiven Endteil (14) umfasst, wobei wenigsten der aktive Endteil aus einem Stammpolymer, das mit einer chemischen Verbindung mit Affinität zu einer Mycobacterium-Komplex-Spezies, einschließlich *Mycobacterium tuberculosis,* funktionalisiert worden ist, für die Entnahme und den wirksamen Nachweis des Vorhandenseins einer *Mycobacterium*-Komplex-Spezies in der Probe hergestellt ist, **dadurch gekennzeichnet, dass** der aktive Endteil die Form eines Schnullers (14B) oder eines Pinsels (14A) mit einem Borstenbündel besitzt und das Stammpolymer mit entweder einem/einer oder mehreren von Concanavalin A und einer positiv geladenen Ammoniumgrundeinheit und einer Kohlenwasserstoffkette funktionalisiert ist, wobei das Concanavalin A und die positiv geladene Ammoniumgrundeinheit und die Kohlenwasserstoffkette an das Stammpolymer kovalent oder elektrostatisch gebunden sind.

2. Die Vorrichtung nach Anspruch 1, wobei das Stammpolymer aus einem oder mehreren von der Gruppe, die aus auf Silicium basierenden Polymeren, Polyester basierenden Polymeren und Polyurethan basierenden Polymeren besteht, ausgewählt ist.

3. Die Vorrichtung nach Anspruch 2, wobei die Kohlenwasserstoffkette eine C₁₀-, C₁₂- oder C₁₄-Kohlenwasserstoffkette ist.

4. Ein Verfahren für die Entnahme einer *Mycobacterium-*Komplex-Spezies, die in einer biologischen Probe von der Mundhöhle eines Patienten vorhanden ist, welches das:
- Bereitstellen eines Stammpolymers in der Vorrichtung (10) durch einen Grundkörper mit einem distalen Ende und einem aktiven Endteil (14), wobei wenigstens der aktive Endteil die Form eines Schnullers (14B) oder eines Pinsels (14A) mit einem Borstenbündel besitzt und aus dem Stammpolymer hergestellt ist, wobei
- das Stammpolymer mit entweder einem/einer oder mehreren von Concanavalin A und einer positiv geladenen Ammoniumgrundeinheit und einer Kohlenwasserstoffkette funktionalisiert ist, wobei das Concanavalin A und die positiv geladene Ammoniumgrundeinheit und die Kohlenwasserstoffkette mit Affinität zu der *Mycobacterium*-Komplex-Spezies kovalent oder elektrostatisch an das Stammpolymer gebunden sind,
- In-Berührung-Bringen des funktionalisierten Polymers mit der Innenseite der Mundhöhle des Patienten und
- Entfernen dieses funktionalisierten Polymers aus der Mundhöhle des Patienten
umfasst.

5. Das Verfahren nach Anspruch 4, wobei das Stammpolymer aus einem oder mehreren von der Gruppe, die aus auf Silicium basierenden Polymeren, Polyester basierenden Polymeren und Polyurethan basierenden Polymeren besteht, ausgewählt ist.

6. Das Verfahren nach Anspruch 5, wobei die Kohlenwasserstoffkette eine C₁₀-, C₁₂- oder C₁₄-Kohlenwasserstoffkette ist.

7. Die Vorrichtung oder das Verfahren nach einem der vorhergehenden Ansprüche, wobei dem Stammpolymer ein Geschmacksstoff hinzugefügt worden ist, um die Entnahme der biologischen Probe für den Patienten angenehmer zu gestalten.

8. Anwendung der Vorrichtung oder des Verfahrens nach einem der vorhergehenden Ansprüche zum Entnehmen einer biologischen Probe mit einer *Mycobacterium-*Komplex-Spezies von der Mundhöhle eines Patienten, um eine Tuberkulose zu diagnostizieren.

9. Ein Verfahren für die Entnahme von *Mycobacterium-*Komplex-Spezies, die in einer biologischen Probe vorhanden sind, und Herstellung eines biologischen Materials daraus durch die Vorrichtung nach den Ansprüchen 1 bis 3, welches das:
A. Entnehmen der *Mycobacterium*-Komplex-Spezies nach einem der Ansprüche 4 bis 6, um ein biologisches Material zu bilden, **dadurch gekennzeichnet, dass** das funktionalisierte Polymer ein Aufnahmegerüst bildet, das die *Mycobacterium*-Komplex-Spezies aus der Mundhöhle des Patienten aktiv entnimmt, und
B. Herstellen des biologischen Materials, einschließlich der Schritte des Veränderns der wesentlichen Eigenschaften der Probe bei Anwesenheit des Aufnahmegerüsts, durch gleichzeitiges:
- Zugeben eines Lysepuffers, der ein Solubilisierungsmittel, ein Detergens und vorzugsweise ein Reduktionsmittel enthält, zu der Probe und
- gleichzeitiges physikalisches Behandeln durch Rühren und Erhöhen der Temperatur der biologischen Probe auf über 40 Grad Celsius und bis zu 100 Grad Celsius, vorzugsweise 92 Grad Celsius, um zeitgleich:
- die Koagulation der Probe zu inhibieren,
- die Probe zu lysieren, um das biologische Material aus der Probe freizusetzen und es zugänglich zu machen, und
- mindestens eine Fraktion des biologischen Materials auf dem Aufnahmegerüst aufzunehmen,
umfasst.

10. Das Verfahren nach Anspruch 9, einschließlich des anschließenden Schritts der Entfernung des Aufnahmegerüsts mit der entnommenen speziellen Fraktion des biologischen Materials darauf aus dem Rest der Probe und vorzugsweise des Waschens des Aufnahmegerüsts mit deionisiertem Wasser.

11. Das Verfahren nach Anspruch 10, einschließlich des anschließenden Schritts des Aufbewahrens, Transports oder Vorlegens der biologischen Probe zu einer Analyse.

12. Ein Kit zur Verwendung bei einem Verfahren für die Entnahme von Mycobacterium-Komplex-Spezies, die in einer biologischen Probe vorhanden sind, und Herstellung von biologischem Material daraus, wobei der Kit:
- eine Vorrichtung für die Entnahme der biologischen Probe nach einem der Ansprüche 1 bis 3, wobei die Vorrichtung ein Aufnahmegerüst bildet, das aktiv die biologische Probe aus der Mundhöhle des Patienten entnimmt, und
- einen Behälter, der einen Lysepuffer umfasst, der ein Solubilisierungsmittel, ein Detergens und vorzugsweise ein Reduktionsmittel enthält, um gleichzeitig aktiv die biologische Probe zu lysieren, um sie zugänglich zu machen, die Koagulation der biologischen Probe zu inhibieren und mindestens eine Fraktion des biologischen Materials auf dem Aufnahmegerüst aufzunehmen,
umfasst.

## Revendications

1. Dispositif (10) pour recueillir un échantillon biologique ayant été prélevé dans la cavité buccale d'un patient, ledit dispositif comprenant un corps ayant une extrémité distale et une partie d'extrémité fonctionnelle (14), au moins ladite partie d'extrémité fonctionnelle étant fabriquée à partir d'un polymère apparenté qui a été rendu fonctionnel avec un composé chimique ayant une affinité pour se combiner à des espèces du complexe *Mycobacterium* englobant le *Mycobacterium tuberculosis,* afin de prélever et de détecter fonctionnellement la présence, dans l'échantillon, d'espèces du complexe *Mycobacterium,* **caractérisé en ce que** la partie d'extrémité fonctionnelle est sous la forme d'une tétine (14B) ou d'une brosse (14A) ayant des brins groupés, et le polymère apparenté a été rendu fonctionnel avec l'un ou plusieurs des composés tels que de la concanavaline A et qu'une fraction d'ammonium à charge positive ainsi qu'une chaîne hydrocarbonée, et où la concanavaline A et la fraction d'ammonium à charge positive ainsi que la chaîne hydrocarbonée sont, de manière covalente ou de manière électrostatique, combinées au polymère apparenté.

2. Dispositif selon la revendication 1, dans lequel le polymère apparenté est sélectionné parmi l'un quelconque ou plusieurs des éléments du groupe se composant de polymères à base de silicium, de polymères à base de polyester et de polymères à base de polyuréthane.

3. Dispositif selon la revendication 2, dans lequel la chaîne hydrocarbonée est une chaîne hydrocarbonée en C₁₀, en C₁₂ ou en C₁₄.

4. Procédé pour collecter des espèces du complexe *Mycobacterium,* lesdites espèces étant présentes dans un échantillon biologique ayant été prélevé dans la cavité buccale d'un patient, ledit procédé consistant :
- à fournir un polymère apparenté dans un dispositif (10) comprenant un corps ayant une extrémité distale et une partie d'extrémité fonctionnelle (14), au moins ladite partie d'extrémité fonctionnelle est sous la forme d'une tétine (14B) ou d'une brosse (14A) ayant des brins groupés et est fabriquée à partir du polymère apparenté ;
- le polymère apparenté a été rendu fonctionnel avec l'un ou plusieurs des composés tels que de la concanavaline A et qu'une fraction d'ammonium à charge positive ainsi qu'une chaîne hydrocarbonée, et où la concanavaline A et la fraction d'ammonium à charge positive ainsi que la chaîne hydrocarbonée sont, de manière covalente ou de manière électrostatique, combinées au polymère apparenté, ledit ou lesdits composé(s) ayant une affinité pour se combiner aux espèces du complexe *Mycobacterium* ;
- à placer le polymère ayant été rendu fonctionnel en contact avec les parois intérieures de la cavité buccale du patient ; et
- à enlever de la cavité buccale du patient ledit polymère ayant été rendu fonctionnel.

5. Procédé selon la revendication 4, dans lequel le polymère apparenté est sélectionné parmi l'un quelconque ou plusieurs des éléments du groupe se composant de polymères à base de silicium, de polymères à base de polyester et de polymères à base de polyuréthane.

6. Procédé selon la revendication 5, dans lequel la chaîne hydrocarbonée est une chaîne hydrocarbonée en C₁₀, en C₁₂ ou en C₁₄.

7. Dispositif ou procédé selon l'une quelconque des revendications précédentes, dispositif ou procédé dans lequel une substance aromatisante est ajoutée au polymère apparenté, pour faire en sorte que le prélèvement de l'échantillon biologique soit plus acceptable pour le patient.

8. Utilisation du dispositif ou du procédé selon l'une quelconque des revendications précédentes, pour recueillir, à des fins de diagnostic de la tuberculose, un échantillon biologique ayant été prélevé dans la cavité buccale d'un patient, ledit échantillon contenant des espèces du complexe *Mycobacterium.*

9. Procédé pour collecter des espèces du complexe *Mycobacterium,* lesdites espèces étant présentes dans un échantillon biologique, et pour préparer, avec le dispositif selon l'une quelconque des revendications 1 à 3, un matériau biologique obtenu à partir desdites espèces ayant été collectées, ledit procédé consistant :
A. à collecter les espèces du complexe *Mycobacterium* selon l'une quelconque des revendications 4 à 6, afin de former un matériau biologique **caractérisé en ce que** le polymère ayant été rendu fonctionnel forme un support de capture qui collecte fonctionnellement les espèces du complexe *Mycobacterium* ayant été prélevées dans la cavité buccale du patient ; et
B. à préparer le matériau biologique, ladite préparation comprenant les étapes consistant à modifier les caractéristiques constitutives de l'échantillon en présence du support de capture, lesdites étapes effectuées de façon concomitante consistant :
- à ajouter à l'échantillon un tampon de lyse contenant un agent solubilisant, un détergent et de préférence un agent réducteur ;
- et, en même temps, à traiter physiquement par agitation et par élévation de la température de l'échantillon biologique, ladite température passant à plus de 40 degrés Celsius et allant jusqu'à 100 degrés Celsius, de préférence jusqu'à 92 degrés Celsius, ledit traitement étant mis en œuvre simultanément :
- pour empêcher une coagulation de l'échantillon ;
- pour procéder à la lyse de l'échantillon, afin que le matériau biologique soit libéré de l'échantillon et afin que le matériau biologique soit rendu disponible ; et
- pour capturer, sur le support de capture, au moins une fraction du matériau biologique.

10. Procédé selon la revendication 9, comprenant l'étape suivante consistant à retirer, du reste de l'échantillon, le support de capture qui contient la fraction particulière de matériau biologique ayant été capturée sur ledit support, et consistant, de préférence, à laver le support de capture avec de l'eau désionisée.

11. Procédé selon la revendication 10, comprenant l'étape suivante consistant à stocker, à transporter ou à présenter l'échantillon biologique pour analyse.

12. Kit pour une utilisation dans un procédé de collecte d'espèces du complexe *Mycobacterium,* lesdites espèces étant présentes dans un échantillon biologique, et pour une utilisation dans un procédé de préparation d'un matériau biologique à partir desdites espèces ayant été collectées, ledit kit comprenant :
- un dispositif pour recueillir l'échantillon biologique selon l'une quelconque des revendications 1 à 3, ledit dispositif formant un support de capture qui recueille fonctionnellement l'échantillon biologique ayant été prélevé dans la cavité buccale du patient ; et
- un récipient comportant un tampon de lyse contenant un agent solubilisant, un détergent et de préférence un agent réducteur, pour, de façon simultanée et fonctionnelle, procéder à la lyse de l'échantillon biologique, afin que le matériau biologique soit rendu disponible, pour empêcher une coagulation de l'échantillon biologique et pour capturer, sur le support de capture, au moins une fraction du matériau biologique.
